# EUROPEAN PATENT SPECIFICATION

(11) **EP 4 529 933 B1**
(45) Date of publication and mention of the grant of the patent: **11.02.2026**
(21) Application number: 23845082.9
(22) Date of filing: 05.06.2023
(51) Int. Cl.: A61L 27/38, A61L 27/50, A61L 27/48, A61L 27/56, A61L 27/58

(54) **IN-VIVO RAPID RECELLULARIZATION TISSUE ENGINEERED BLOOD VESSEL AND PREPARATION METHOD THEREFOR**
DURCH GEWEBE MANIPULIERTES BLUTGEFÄSS FÜR SCHNELLE IN-VIVO-REZELLULARISIERUNG UND HERSTELLUNGSVERFAHREN DAFÜR
VAISSEAU SANGUIN MODIFIÉ PAR UN TISSU À RECELLULARISATION RAPIDE IN VIVO ET SON PROCÉDÉ DE PRÉPARATION

(30) Priority: 29.07.2022 CN 202210906816
(43) Date of publication of application: 02.04.2025
(73) Proprietor: Humatrix Medical Technology (Suzhou) Co., Ltd., Suzhou, Jiangsu 215000 (CN)
(72) Inventor: QIU, Xuefeng, Suzhou, Jiangsu 215000 (CN); GUO, Ying, Suzhou, Jiangsu 215000 (CN)
(74) Representative: Bayramoglu et al.
(86) International application number: PCT/CN2023/098423
(87) International publication number: WO 2024/021869

(56) References cited:
- CN-A- 106 267 369
- CN-A- 108 434 519
- CN-A- 111 603 611
- CN-A- 111 714 248
- CN-A- 111 714 706
- CN-A- 113 694 253
- CN-A- 114 949 362
- US-A1- 2006 073 590
- US-A1- 2015 031 131
- US-A1- 2015 031 131
- US-A1- 2019 321 158
- US-A1- 2021 060 208
- SHANNON LM DAHL ET AL: "Readily Available Tissue-Engineered Vascular Grafts", SCIENCE TRANSLATIONAL MEDICINE, vol. 3, no. 68, 2 February 2011 (2011-02-02), pages 68ra9, XP055081045, DOI: 10.1126/scitranslmed.3001426

## Description

### [TECHNICAL FIELD]

The present disclosure relates to the field of medical device technology, and particularly, to a method for preparing a tissue engineering blood vessel for *in vivo* rapid recellularization.

### [BACKGROUND TECHNOLOGY]

Along with social progress and the improvement of living standards, cardiovascular diseases have become the leading cause of death, threatening people's health, and there is an increasing clinical demand for small-diameter artificial blood vessels (less than 6 mm in diameter). At present, China sees about 870,000 peripheral artery disease surgeries each year, while the United States sees over 100,000 peripheral artery disease surgeries each year. In China, 150,000 vascular trauma surgeries are performed annually, while in the United States, 73,000 are performed. In China, the number of coronary artery bypass surgeries performed per year exceeds 50,000 and is expected to reach 150,000 within the next decade, and the counterpart in the United States is 500,000, with each patient requiring an average of about 2.5 bypass blood vessels. Autologous vein grafts have to be collected surgically, which may easily lead to extended surgery time, graft incision infection, and prolonged ICU stays. Additionally, in many patients, the great saphenous vein does not meet the clinical requirements. Each year, about over 3.5 million hemodialysis treatments are given. The patients require a safe, reusable arteriovenous (AV) channel as dialysis access. According to the Chinese National Renal Data System (CNRDS), the rate of hemodialysis is increasing annually by 13-14%. In China, there are over 120 million patients with chronic kidney disease, with more than 800,000 registered for long-term dialysis in 2020, and projections indicate that this number will exceed 2.3 million by 2030. Of these, 87% use arteriovenous fistulas, 10% use central venous catheters, and about 1% use artificial vascular access. In the United States, about 600,000 hemodialysis treatments are given to patients with chronic diseases each year. Of these, 65% use arteriovenous fistulas, 15% use central venous catheters, and 20% use artificial vascular access, with the use of artificial vascular access on the rise. Patients undergoing arteriovenous fistula surgery need to wait for 3-6 months before the arteriovenous fistula is available as dialysis access. However, for about one-third to half of these patients, the arteriovenous fistula becomes unusable after the 3- to 6-month waiting period, and such an access may degenerate due to its susceptibility to infection, thrombosis, or aneurysm formation and may thus be clinically unusable. Central venous catheters can only be used as temporary dialysis access, with an annual infection rate of up to 200% in patients. Conventional expanded polytetrafluoroethylene (ePTFE) artificial blood vessels are prone to infection, with a 2-year secondary patency rate of about 50% and a very low 5-year patency rate.

Existing commercially available small-diameter artificial blood vessels (such as those made of Teflon and polyurethane or decellularized blood vessels from allogeneic or xenogeneic sources) feature low patency rates and are prone to thrombosis and infection, which affect clinical outcomes. The primary reason is the failure in effective vascular wall recellularization, including endothelialization and vascular smooth muscle cell recellularization. Therefore, there is a pressing clinical need to develop high-performance, clinically translatable, small-diameter artificial blood vessels.

Conventional tissue engineering techniques require several months to construct the small-diameter tissue engineering vascular graft (TEVG). These processes are usually cost-inefficient, with uncertain safety profiles for use in humans. Moreover, they may require special methods for storage and transportation before surgery, making these techniques unsuitable for widespread clinical use. The *in situ* tissue engineering technology avoids complex processes such as *in vitro* cell isolation, culture, and cell-scaffold integration in the conventional tissue engineering techniques through direct implantation of a tissue engineering blood vessel into the body, where it recruits endogenous cells to regenerate the vessel *in situ.* Moreover, this technology meets the clinical need for "on-demand availability", offering significant advantages for promoting clinical applications. However, current research on *in situ* tissue engineering faces notable challenges, such as insufficient vascular endothelialization, which leads to thrombosis, and insufficient vascular wall recellularization with vascular smooth muscle cells, which causes infection or aneurysm formation. These issues result in low long-term patency rates, significantly limiting the clinical translation.

CN 111 714 706 discloses a stent which is formed by spinning degradable polymer fibers in combination with a growth factor. The fiber diameter from which the inner layer of the stent is formed is larger as compared to the diameter of the fibers of the outer layer. Cells are seeded and cultured onto the stent under increasing pressure conditions so as to secrete an extracellular matrix and form an artificial blood vessel. This blood vessel is then decellularized whereby the ECM remains intact.

### [CONTENT OF THE INVENTION]

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

To solve the shortcomings in the prior art, such as the recellularization failure or slow recellularization on artificial blood vessels, low patency rates, susceptibility to infection, and high degeneration rates, the present disclosure provides a method for preparing a tissue engineering blood vessel for *in vivo* rapid recellularization. By employing biomaterial modification and tissue engineering biomimetic techniques, the present disclosure significantly enhances the adhesion, growth, and proliferation of seed cells on degradable scaffold materials. During culture, the degradable polymer composite scaffold completely degrades, reducing the culture period. Through decellularization techniques, a decellularized matrix-based tissue engineering blood vessel with a special biomimetic extracellular matrix 3D structure is acquired. The biomimetic extracellular matrix structure allows the blood vessel, when implanted into the body, to realize rapid *in vivo* recellularization, construct a new blood vessel *in situ* in the body, and improve the blood vessel's performance. The present disclosure meets the clinical need for "on-demand availability", facilitating clinical translation.

To achieve the above objectives, provided is a method for preparing a tissue engineering blood vessel for *in vivo* rapid recellularization, comprising:
S1: dissolving polyglycolic acid and polyethylene glycol separately in an organic solvent to prepare spinning solutions, and preparing a tubular polymer composite scaffold having a special tube wall structure by electrospinning from the two spinning solutions in a certain ratio, wherein the mass ratio of the polyglycolic acid spinning solution to the polyethylene glycol spinning solution for an inner tube wall of the tubular polymer composite scaffold is higher than the mass ratio of the polyglycolic acid spinning solution to the polyethylene glycol spinning solution for an outer tube wall of the tubular polymer composite scaffold;
S2: seeding a seed cell on the tubular polymer composite scaffold having the special tube wall structure, and culturing for 3 weeks to 3 months in a certain perfusion culture condition simulating an arterial pulsatile flow in a body to give an engineering blood vessel composed of the seed cell and an extracellular matrix; and
S3: decellularizing the engineering blood vessel to remove vascular cell components, so as to give the tissue engineering blood vessel composed of the extracellular matrix.

Preferably, in step S1, the mass ratio of the polyglycolic acid spinning solution to the polyethylene glycol spinning solution for the inner tube wall of the tubular polymer composite scaffold is 15:1-50:1, and the mass ratio of the polyglycolic acid spinning solution to the polyethylene glycol spinning solution for the outer tube wall of the tubular polymer composite scaffold is 5:1-15:1.

Preferably, the tubular polymer scaffold has an inner diameter of 1-35 mm, a tube wall thickness of 0.01-3.5 mm, a length of 0.5-120 cm, and a tube wall porosity of 85-99.9%.

Preferably, the culture condition simulating the arterial pulsatile flow in the body in step S2 is as follows:
firstly, a static *in vitro* culture of 1-10 days; secondly, a culture of 6-8 days in an arterial pulsatile flow at a pressure of 10-90 mmHg; thirdly, a culture in an arterial pulsatile flow at an increased pressure of 90-10 mmHg; and finally, a culture of one week in an arterial pulsatile flow at an increased pressure of 110-160 mmHg.

Preferably, the decellularization in step S3 comprises the following steps:
treating the cultured tubular tissue in a prepared decellularization reagent for 1-3 h at room temperature, and then replacing the decellularization reagent 3-6 times, once every 1-3 h, to give the decellularized tissue engineering blood vessel;
the decellularization reagent is prepared from 3-[(3-cholamidopropyl)-diethylammonio]-propanesulfonate, disodium edetate (EDTA-2Na), NaCl, NaOH, and sterile deionized water.

Preferably, the seed cells are uniformly seeded on the tubular polymer composite scaffold at 0.1-3.0 × 10⁶ cells/centimeter.

Preferably, the seed cells include, but are not limited to, vascular smooth muscle cells or fibroblasts from an adult human or mammal, vascular smooth muscle cells from an umbilical vein/umbilical artery, and vascular smooth muscle cells or fibroblasts differentiated from a mesenchymal stem cell and an induced pluripotent stem cell.

Preferably, the seed cells are primary to 10th-passage cells, preferably primary to 4th-passage cells, from a single donor or a cell bank.

In general, compared to the prior art, the above technical solutions contemplated by the present disclosure can achieve the following beneficial effects:
(1) According to the tissue engineering blood vessel for *in vivo* rapid recellularization prepared by the method as defined in the appended claims, the degradable polymer composite scaffold with the special tube wall structure is prepared from polyglycolic acid and polyethylene glycol in a certain ratio by electrospinning. The fibers on the outer side of the vascular scaffold contain a higher proportion of polyethylene glycol than the fibers on the inner side of the tube wall. When the seed cells are seeded on the scaffold and subjected to *in vitro* perfusion culture in a certain condition that simulates the arterial pulsatile flow in the body, the degradable polymer scaffold completely degrades, and an engineering blood vessel composed of the seed cells and the extracellular matrix is acquired. Then the engineering blood vessel is decellularized to give the tissue engineering blood vessel consisting of the extracellular matrix with a biomimetic structure. The decellularized matrix-based tissue engineering blood vessel has a special biomimetic extracellular matrix 3D structure. The extracellular matrix with the biomimetic structure allows the decellularized matrix-based blood vessel, after being implanted into the body, to be rapidly recellularized and secrete a new extracellular matrix, facilitating the self-repair and renewal of the vascular wall's extracellular matrix to resist infection. Meanwhile, the lumen of the decellularized matrix-based blood vessel is rapidly endothelialized (1 week to 1 month), improving the patency rate of the blood vessel. The blood vessel of the present disclosure is superior to conventional artificial blood vessels.
(2) According to the tissue engineering blood vessel for *in vivo* rapid recellularization prepared by the method as defined in the appended claims, the degradable polymer composite scaffold, compared to composite scaffold fibers and pure polyglycolic acid fibers in *in vitro* culture, can promote cell adhesion, increasing the success rate of cell seeding. During culture, particularly in the early stages of culture, the tube wall structure, compared to pure polyglycolic acid materials, allows the seeded seed cells to adhere to scaffold fibers containing polyethylene glycol, so that the cells will not easily fall off. In the static and dynamic perfusion culture processes, the outer side of the tube wall degrades first due to the higher proportion of polyethylene glycol, resulting in pores that facilitate the migration and proliferation of the seed cells on the tube wall toward the inner side of the tube wall. The inner side of the tube wall degrades continuously and slowly due to the high proportion of polyglycolic acid, thereby maintaining the mechanical performance of the tube wall of the scaffold material. The present disclosure overcomes the shortcomings of conventional tissue engineering techniques, such as insufficient cell viability and slow growth after seed cells are seeded on the scaffold material.
(3) According to the tissue engineering blood vessel for *in vivo* rapid recellularization prepared by the method as defined in the appended claims, a combination of static culture and perfusion culture in a condition that simulates the arterial pulsatile flow in the body is used to promote the uniform growth, migration, and proliferation of seed cells on the fiber surfaces of the inner and outer sides of the vascular scaffold material, accelerating the synthesis of extracellular matrix proteins. The proteins in the extracellular matrix secreted by the cells on the scaffold include, but are not limited to, collagen I and collagen III. Thus, the inner wall structure of the lumen is smooth and uniform, and the vascular wall does not easily break and has good mechanical performance. This significantly increases the success rate of *in vitro* blood vessel culture and reduces culture costs. The present disclosure has more advantages of being commercialized.
(4) According to the tissue engineering blood vessel for *in vivo* rapid recellularization prepared by the method as defined in the appended claims, the seed cells include, but are not limited to, adult human vascular smooth muscle cells, human fibroblasts, human umbilical artery and human umbilical vein vascular smooth muscle cells, and human vascular smooth muscle cells and human fibroblasts differentiated from human mesenchymal stem cells including human adipose-derived stem cells and human induced pluripotent stem cells, vascular smooth muscle cells and fibroblasts from other adult mammalian sources (including pigs, cattle, sheep, etc.), and vascular smooth muscle cells and fibroblasts differentiated from mesenchymal stem cells including adipose-derived stem cells and induced pluripotent stem cells. The sources are diverse and unrestricted, thus facilitating large-scale, commercial cultures.

### [DESCRIPTION OF THE DRAWINGS]

FIG. 1 shows a schematic diagram of a tubular scaffold provided by an example of the present disclosure.
FIG. 2 shows a SEM topographic image of the outer wall of a tubular scaffold provided in an example of the present disclosure.
FIG. 3 shows a SEM topographic image of the inner wall of a tubular scaffold provided in an example of the present disclosure.
FIG. 4 shows a SEM topographic image of a cross-section of a tubular scaffold provided in an example of the present disclosure.
FIG. 5 shows a SEM topographic image of a cross-section of a tubular scaffold provided in an example of the present disclosure.

### [SPECIFIC IMPLEMENTATIONS]

To make the objectives, technical solutions, and advantages of the present disclosure more clear, the present disclosure is further described in detail below with reference to the drawings and examples. It will be appreciated that the specific examples described herein are merely illustrations of the present disclosure and do not limit the present disclosure. In addition, the technical features described below in the embodiments of the present disclosure can be combined with each other as long as they do not conflict with each other.

In the present disclosure, a degradable polymer composite scaffold with a special tube wall structure is constructed from polyglycolic acid and polyethylene glycol by electrospinning, with the fibers on the outer side of the vascular scaffold containing a higher proportion of polyethylene glycol. When seed cells are seeded on the scaffold and subjected to *in vitro* perfusion culture in a certain condition that simulates the arterial pulsatile flow in the body, the degradable polymer degrades, and an engineering blood vessel composed of the seed cells and an extracellular matrix is acquired. The engineering blood vessel is further decellularized to give a tissue engineering blood vessel composed of the extracellular matrix with a biomimetic structure. The method specifically comprises the following steps:
S1: Preparation of tubular polymer composite scaffold with special tube wall structure

Polyglycolic acid and polyethylene glycol are separately dissolved in an organic solvent to give uniform, stable spinning solutions. The two spinning solutions are used for electrospinning in a certain ratio and received by a Teflon-coated metal rod, so as to give an oriented fiber tubular polymer composite scaffold having a special tube wall structure. The mass ratio of the polyglycolic acid spinning solution to the polyethylene glycol spinning solution for an inner tube wall of the tubular polymer composite scaffold is higher than the mass ratio of the polyglycolic acid spinning solution to the polyethylene glycol spinning solution for an outer tube wall of the tubular polymer composite scaffold.

Specifically, the spinning solutions have a polyglycolic acid concentration of 1-500 mg/mL and a polyethylene glycol concentration of 1-500 mg/mL; the mass ratio of the polyglycolic acid spinning solution to the polyethylene glycol spinning solution for the inner tube wall of the tubular polymer composite scaffold is 15:1-50:1, and the mass ratio of the polyglycolic acid spinning solution to the polyethylene glycol spinning solution for the outer tube wall of the tubular polymer composite scaffold is 5:1-15:1. The electrospinning fibers have a diameter of 5 nanometers to 50 micrometers; the organic solvent includes, but is not limited to, one or more of hexafluoroisopropanol, trichloromethane, and the like.

In the preparation of tissue engineering blood vessels, scaffold preparation is crucial. In the present application, two polymers, polyglycolic acid and polyethylene glycol, are selected as spinning solutions to prepare the degradable polymer composite scaffold with the special tube wall structure. The fibers on the outer side of the vascular scaffold contain a higher proportion of polyethylene glycol. In the early stages of culture, when the tube-wall cells have not yet fully grown and proliferated, the polyglycolic acid fibers can maintain the scaffold's mechanical performance. The polyethylene glycol fibers can promote the adhesion, migration, and proliferation of seed cells in the early stages of culture and ensure sufficient cell growth on the tube wall in static and pulsatile flow conditions. The two composite material fiber structures gradually degrade during the culture and cell growth, and the polyethylene glycol fibers degrade faster than polyglycolic acid. Some of the fibers on the outer side of the tube wall degrade preferentially due to the higher proportion of polyethylene glycol, resulting in pores that facilitate the migration and proliferation of the seed cells on the tube wall toward the inner side of the tube wall. The inner side of the tube wall degrades continuously and slowly due to the high proportion of polyglycolic acid, thereby maintaining the mechanical performance of the tube wall of the scaffold material. This further facilitates the migration and growth of cells in the middle and inner layers without affecting the mechanical performance of the tube wall of the composite fiber scaffold. Consequently, the vascular cells secrete more extracellular matrix more rapidly, improving the mechanical performance of the wall of the cultured blood vessel, significantly increasing the success rate of culture, and reducing the culture time.

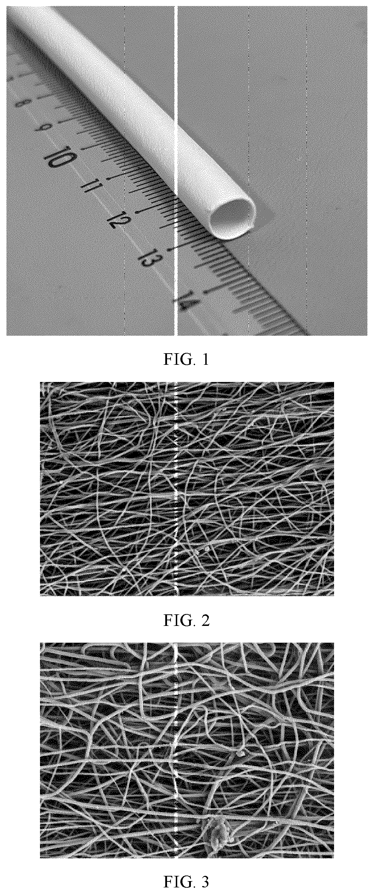

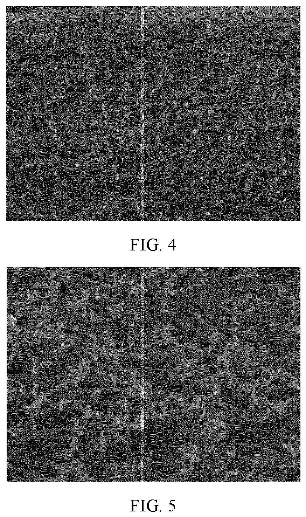

FIG. 2 and FIG. 3 show the SEM topographic images of the outer wall and inner wall of a tubular scaffold provided in an example of the present disclosure, respectively. It can be seen from the images that the outer-wall fibers of the tubular scaffold are significantly thinner than the inner-wall fibers. This is because the inner wall has a higher proportion of polyglycolic acid than the outer wall. During culture, the outer-wall fibers degrade more quickly, promoting cell growth, while the inner-wall fibers degrade slowly, maintaining the mechanical performance of the tube wall. FIG. 4 and FIG. 5 show the SEM topographic images of the cross-sections of a tubular scaffold provided in an example of the present disclosure. It can be seen that the tube wall is a highly porous structure.

### S2: In vitro culture of engineering blood vessel

Seed cells are seeded on the tubular polymer composite scaffold, cultured statically for 1-10 days, and then cultured for 3 weeks to 3 months in a certain perfusion culture condition simulating the arterial pulsatile flow in the body to allow the seed cells to adequately grow and proliferate on the composite scaffold. The tube-wall polymer gradually degrades, while the vascular cells secrete the extracellular matrix to gradually enhance the mechanical performance of the tube wall. Finally, an engineering blood vessel composed of the seed cells and the extracellular matrix is acquired.

Specifically, the culture condition simulating the arterial pulsatile flow in the body is as follows: firstly, a static in vitro culture of 1-10 days; secondly, a culture of 6-8 days in an arterial pulsatile flow at a pressure of 10-90 mmHg; thirdly, a culture in an arterial pulsatile flow at an increased pressure of 90-110 mmHg; and finally, after the scaffold material completely degrades, a culture of one week in an arterial pulsatile flow at an increased pressure of 110-160 mmHg to significantly improve the mechanical performance of the vascular wall. The *in vitro* culture period is 3 weeks to 3 months, preferably 6-12 weeks; all dynamic cultures are perfusion cultures.

Seed cell seeding is one of the key steps in tissue engineering blood vessel preparation. In the present application, the seed cells include, but are not limited to, adult human vascular smooth muscle cells, human fibroblasts, human umbilical artery and human umbilical vein vascular smooth muscle cells, and human vascular smooth muscle cells and human fibroblasts differentiated from human mesenchymal stem cells including human adipose-derived stem cells and human induced pluripotent stem cells, vascular smooth muscle cells and fibroblasts from other adult mammalian sources (including pigs, cattle, sheep, etc.), and vascular smooth muscle cells and fibroblasts differentiated from mesenchymal stem cells including adipose-derived stem cells and induced pluripotent stem cells.

More specifically, the cells are primary to 10th-passage cells, preferably primary to 4th-passage cells, from one or more donors or a cell bank and are cultured *in vitro* for 3 weeks to 3 months, most preferably for 6-12 weeks. The cell culture medium contains a high content of sugar, insulin (0.1-5.0 mg/mL), the growth factors bFGF and/or EGF, penicillin (1-300 U/mL), streptomycin (1-500 mg/mL), and vitamin C (0.5-800 mg/mL), and may be, for example, DMEM. In weeks 0-4 of the culture, 5-50% human or mammal serum is added to the culture medium. On the following days, upon the culture medium replacements once every 3-7 days, the human or mammal serum concentration is reduced by 1-20%. The procedures continue until the culture is finished.

More specifically, the cells are uniformly seeded on the composite scaffold at 0.1-3.0 × 10⁶ cells per centimeter, and the optimal seeding density is 1.0-2.0 × 10⁶ cells/centimeter.

The degradable polymer composite scaffold fibers gradually degrade in the culture condition described above, and an engineering blood vessel consisting of the seed cells and the extracellular matrix secreted by the seed cells is acquired. In the present application, by combining static culture with culture in a condition simulating the arterial pulsatile flow in the body, the uniform growth and proliferation of the seed cells on the inner and outer sides of the vascular scaffold material and thus the synthesis of the extracellular matrix are promoted. The extracellular matrix secreted by the cells on the scaffold includes, but is not limited to, collagen I fiber and collagen III fiber, ensuring that the tube wall has good mechanical properties, including a burst pressure of 1000-5000 mmHg, a suture tension of 100-250 g, and an elastic modulus of 0.5-3.0 MPa. Particularly, during early dynamic culture, the tube wall does not easily break, and the structure of the inner wall of the lumen is smooth. The success rate of *in vitro* culture has increased from 80-85% of conventional techniques to 95-100%. Therefore, the present disclosure has more advantages for commercialization.

### S3: Decellularization

The engineering blood vessel is decellularized to give an extracellular matrix 3D structure that is looser than arterial vessel walls in the body, which is conducive to vascular wall recellularization after the implantation of the blood vessel into the body and ensures that the vascular wall composed of the extracellular matrix has excellent mechanical performance.

The decellularized tissue engineering blood vessel is mainly composed of the extracellular matrix and may comprise trace amounts of residual polyglycolic acid and/or polyethylene glycol. The proteins of extracellular matrix of the vascular wall include, but are not limited to, collagen I and collagen III. The amount of cells in the vascular wall is less than 1-2%. If the blood vessel is completely decellularized, the cell DNA content is less than 0.1%-0.001%. Meanwhile, the decellularization does not affect the mechanical performance of the blood vessel. Unlike conventional tissue engineering blood vessels that require a prolonged culture period and special methods for preservation and transportation, the blood vessel of the present disclosure can be preserved at room temperature for a long time, meeting the clinical need for "on-demand availability".

The decellularization may be conducted using a chemical reagent, electric treatment, etc. In one preferred example, the decellularization specifically comprises the following steps:
(1) preparation of decellularization reagent: mixing 3-[(3-cholamidopropyl)-diethylammonio]-propanesulfonate, EDTA-2Na, NaCl, and NaOH with sterile deionized water to prepare a solution, with their molar concentrations being 1-40 mmol/L, 0.5-50 mmol/L, 0.01-1.0 mmol/L, and 0.1-10.0 mol/L, respectively, wherein the molar concentration of 3-[(3-cholamidopropyl)-diethylammonio]-propanesulfonate is preferably 10 mmol/L, the molar concentration of EDTA-2Na₂ is preferably 30 mmol/L, the molar concentration of NaCl is preferably 0.15 mmol/L, and the molar concentration of NaOH is preferably 2 mol/L;
(2) decellularization: treating the engineering blood vessel in the decellularization reagent for 1-5 h at room temperature, and then replacing the decellularization reagent 2-8 times, preferably 3 times, once every 1-5 h, to give the decellularized matrix-based tissue engineering blood vessel;
   and
(3) washing: thoroughly washing the decellularized matrix-based tissue engineering blood vessel obtained in step (2) with a sterile PBS solution to remove decellularization reagent residues.

The method for preparing a tissue engineering blood vessel for rapid recellularization as provided by the present invention is further described in detail below using different specific examples. The specific examples are as follows:

### Example 1

### S1: Preparation of tubular polymer composite scaffold

Polyglycolic acid and polyethylene glycol were separately dissolved in hexafluoroisopropanol solvent to prepare uniform, stable spinning solutions. The tube wall of the scaffold was 480 µm thick. During the spinning, spinning solutions of two mass ratios were used. The mass ratio of polyglycolic acid spinning solution to polyethylene glycol spinning solution was 30:1 for the 240-micrometer thick tube wall on the inner side, and the mass ratio of polyglycolic acid spinning solution to polyethylene glycol spinning solution was 5:1 for the 240-micrometer thick tube wall on the outer side. An oriented fiber tubular polymer composite scaffold was obtained.

### S2: Seeding of seed cells on tubular polymer composite scaffold and in vitro perfusion culture

Human aortic vascular smooth muscle cells at the 3rd passage were resuspended in a low sugar DMEM culture medium containing 10% FBS, seeded on the tubular polymer composite scaffold at a density of 1.0 × 10⁶ cells/cm, statically cultured for 5 days in a 37 °C, 5% CO₂ incubator, then cultured for 1 week in a pulsatile flow at 30 mmHg, cultured for 1 week in a pulsatile flow at 60 mmHg, cultured for 1 week at 90 mmHg, and cultured for 3 weeks at 120 mmHg to give an engineering blood vessel, with the culture time being less than 7 weeks.

### S3: Decellularization

### (1) Preparation of decellularization reagent

| Reagent | Molar concentration | Mass |
|---|---|---|
| 3-[(3-Cholamidopropyl)-diethylammonio ]-propanesulfonate | 10 mM | 3.075 g |
| EDTA-2Na | 30 mM | 5,58 g |
| NaCl | 0.15 mM | 3.6 g |
| NaOH | 2 M | 58.44 g |
| Sterile water | | 500 ml |

According to the formula shown in the table above, exact amounts of the reagents were added to a clean wide-mouth glass flask. The flask was shaken on a shaker for 2-3 h, until the solution became clear, to fully dissolve the reagents, and thus the decellularization reagent was prepared. The decellularization reagent was preserved at 4 °C before use.

The engineering blood vessel was placed into a sterile centrifuge tube and perfused at room temperature with the decellularization reagent for 5 h. After the decellularization was complete, the blood vessel was perfused and washed with a sterile PBS solution for 3 h to remove the decellularization reagent. Finally, the resulting decellularized tissue engineering blood vessel was placed in normal saline at 4 °C for preservation before use.

### Example 2

### S1: Preparation of tubular polymer composite scaffold

Polyglycolic acid and polyethylene glycol were separately dissolved in hexafluoroisopropanol solvent to prepare uniform, stable spinning solutions. The tube wall of the scaffold was 480 µm thick. During the spinning, spinning solutions of two mass ratios were used. The mass ratio of polyglycolic acid spinning solution to polyethylene glycol spinning solution was 15:1 for the 240-micrometer thick tube wall on the inner side, and the mass ratio of polyglycolic acid spinning solution to polyethylene glycol spinning solution was 5:1 for the 240-micrometer thick tube wall on the outer side. An oriented fiber tubular polymer composite scaffold was obtained.

### S2: Seeding of seed cells on tubular polymer scaffold and in vitro perfusion culture

Human umbilical vein vascular smooth muscle cells at the 3rd passage were resuspended in a low sugar DMEM culture medium containing 10% FBS, seeded on the tubular polymer composite scaffold at a density of 0.8 × 10⁶ cells/cm, statically cultured for 5 days in a 37 °C, 5% CO₂ incubator, then cultured for 1 week in a pulsatile flow at 30 mmHg, cultured for 1 week in a pulsatile flow at 60 mmHg, cultured for 1 week at 90 mmHg, and cultured for 2 weeks at 120 mmHg to give an engineering blood vessel, with the culture time being not greater than 8 weeks.

### S3: Decellularization

The procedures for decellularization are the same as those in Example 1. Please refer to Example 1.

### Example 3

### S1: Preparation of tubular polymer composite scaffold

Polyglycolic acid and polyethylene glycol were separately dissolved in hexafluoroisopropanol solvent to prepare uniform, stable spinning solutions. The tube wall of the scaffold was 480 µm thick. During the spinning, spinning solutions of two mass ratios were used. The mass ratio of polyglycolic acid spinning solution to polyethylene glycol spinning solution was 30:1 for the 240-micrometer thick tube wall on the inner side, and the mass ratio of polyglycolic acid spinning solution to polyethylene glycol spinning solution was 5:1 for the 240-micrometer thick tube wall on the outer side. An oriented fiber tubular polymer composite scaffold was obtained.

### S2: Seeding of seed cells on tubular polymer scaffold and in vitro perfusion culture

Human fibroblasts at the 3rd passage were resuspended in a low sugar DMEM culture medium containing 10% FBS, seeded on the tubular polymer composite scaffold at a density of 0.5 × 10⁶ cells/cm, statically cultured for 5 days in a 37 °C, 5% CO₂ incubator, then cultured for 1 week in a pulsatile flow at 30 mmHg, cultured for 1 week in a pulsatile flow at 60 mmHg, cultured for 1 week at 90 mmHg, and cultured for 2 weeks at 120 mmHg to give an engineering blood vessel, with the culture time being less than 6 weeks.

### S3: Decellularization

The procedures for decellularization are the same as those in Example 1. Please refer to Example 1.

### Example 4

### S1: Preparation of tubular polymer composite scaffold

Polyglycolic acid and polyethylene glycol were separately dissolved in hexafluoroisopropanol solvent to prepare uniform, stable spinning solutions. The tube wall of the scaffold was 480 µm thick. During the spinning, spinning solutions of two mass ratios were used. The mass ratio of polyglycolic acid spinning solution to polyethylene glycol spinning solution was 50:1 for the 240-micrometer thick tube wall on the inner side, and the mass ratio of polyglycolic acid spinning solution to polyethylene glycol spinning solution was 5:1 for the 240-micrometer thick tube wall on the outer side. An oriented fiber tubular polymer composite scaffold was obtained.

### S2: Seeding of seed cells on tubular polymer scaffold and in vitro perfusion culture

Vascular smooth muscle cells differentiated from human induced pluripotent stem cells, at the 3rd passage, were resuspended in a low sugar DMEM culture medium containing 10% FBS, seeded on the tubular polymer composite scaffold at a density of 1.0 × 10⁶ cells/cm, statically cultured for 5 days in a 37 °C, 5% CO₂ incubator, then cultured for 1 week in a pulsatile flow at 30 mmHg, cultured for 1 week in a pulsatile flow at 60 mmHg, cultured for 1 week at 90 mmHg, and cultured for 3 weeks at 120 mmHg to give an engineering blood vessel, with the culture time being less than 7 weeks.

### S3: Decellularization

The procedures for decellularization are the same as those in the above examples. Please refer to Example 1.

### Example 5

Before and after decellularization, the engineering blood vessels in Examples 1-4 were subjected to burst pressure, suture tension, maximum tensile stress, and maximum tensile strain tests, and the test results were compared. There were no statistically significant differences between the test results before and after decellularization. The specific testing method and results are as follows:

### (1) Burst pressure test

The burst pressure test was conducted on an in-house pressure measuring system equipped with a precise pressure gauge. The pressure measuring system was filled with a PBS buffer, and a test sample with a length of 8 cm was connected to the pressure measuring system. The pressure of the pressure measuring system was gradually increased until a sudden pressure drop was detected and a rupture in the vessel was observed by reviewing the video. The maximum pressure was recorded and used as the burst pressure.

### (2) Suture tension test

The suture tension refers to the force when sutures tear the tissue. In this test, the suture tension was measured according to the ANSI/AAMI/ISO 7198 standard. A test sample with a length of 2 cm was prepared. One end of the vessel was sewn with 6-0 Prolene sutures at 2 mm from the edge, with a total of 3 stitches placed at 120-degree intervals. Each suture was tied independently to form a loop. The end of the vessel without sutures and one of the sutures were fixed to a universal tensile testing system. The stretching speed was adjusted to 30 mm/min, and the maximum force was recorded. The test was repeated on the other two sutures. The average of the 3 test results is the suture tension.

### (3) Maximum tensile stress and maximum tensile strain

The test tissue vessel was cut into 5-mm wide rings, and their thickness and diameter were measured and recorded. The rings were hung by the two ends on paper clips, and then 2 paper clips were fixed to a universal tensile testing system. The initial stretch length was about 10% of the breaking length. The stretching speed was adjusted to 30 mm/min. The test was continued until the blood vessel ring was broken, and the maximum tensile stress and the maximum tensile strain were obtained.

### Before decellularization:

**Table 1. Results of the mechanical performance tests for tissue engineering blood vessels in Examples 1-4 before decellularization**

| Sample (n = 8) | Burst pressure (mmHg) | Suture tension (N) | Maximum tensile stress (MPa) | Maximum tensile strain (%) |
|---|---|---|---|---|
| Example 1 | 3170±92 | 1.50±0.23 | 3.56±0.25 | 27.30±3.55 |
| Example 2 | 3520±113 | 1.93±0.04 | 4.47±0.39 | 25.07±5.23 |
| Example 3 | 3406±50 | 1.81±0.18 | 3.16±0.53 | 36.49±7.03 |
| Example 4 | 2917±170 | 1.27±0.12 | 2.41±0.36 | 22.61±2.87 |

### After decellularization:

**Table 2. Results of the mechanical performance tests for tissue engineering blood vessels in Examples 1-4 after decellularization**

| Sample (n = 8) | Burst pressure (mmHg) | Suture tension (N) | Maximum tensile stress (MPa) | Maximum tensile strain (%) |
|---|---|---|---|---|
| Example 1 | 3025±117 | 1.33±0.12 | 3.29±0.18 | 29.05±4.97 |
| Example 2 | 3397±85 | 1.80±0.19 | 4.20±0.11 | 23.60±3.69 |
| Example 3 | 3160±170 | 1.54±0.07 | 2.98±0.26 | 35.92±6.53 |
| Example 4 | 2801±142 | 1.23±0.20 | 2.29±0.17 | 20.01±3.52 |

According to the data from Table 1 and Table 2, the tissue engineering blood vessels prepared according to the method of the present invention possess good biocompatibility, good mechanical performance, ease to suture, and tolerance to high burst pressures and tensile forces, making them suitable for clinical applications. In addition, the decellularization method of the present disclosure has little impact on the mechanical performance of the vascular walls of the engineering blood vessels.

In conclusion, the method for preparing a tissue engineering blood vessel for *in vivo* rapid recellularization provided by the present disclosure has the advantages of:
(1) on-demand availability: the present disclosure can avoid prolonged periods for blood vessel culture and eliminate the need to surgically acquire seed cells from the patient;
(2) regenerative and self-repair capabilities: once the blood vessel is implanted into the body, the patient's own cells can reconstruct the decellularized matrix-based blood vessel, promoting vascular regeneration and enabling self-repair;
(3) no immunological rejection: No donor tissue matching or lifelong use of immunosuppressants to suppress immune responses is required;
(4) resistance to infection: the patient's own cells reconstruct the vascular lumen and wall, and the extracellular matrix self-renews and can automatically restore its structure after vascular puncture, thereby resisting infection and preventing vascular degeneration; and
(5) high patency rates: when used as a dialysis channel implanted in the body, the tissue engineering blood vessel can be immediately punctured and used without waiting, and may also resist thrombosis and feature durability and long-term use.

It will be appreciated that the above examples are merely illustrative of the technical solutions of the present disclosure and are not intended to limit them.

## Claims

1. A method for preparing a tissue engineering blood vessel for *in vivo* rapid recellularization, comprising:
S1: dissolving polyglycolic acid and polyethylene glycol separately in an organic solvent to prepare spinning solutions, and preparing a tubular polymer composite scaffold by electrospinning from the two spinning solutions in a certain ratio, wherein the mass ratio of the polyglycolic acid spinning solution to the polyethylene glycol spinning solution for an inner tube wall of the tubular polymer composite scaffold is higher than the mass ratio of the polyglycolic acid spinning solution to the polyethylene glycol spinning solution for an outer tube wall of the tubular polymer composite scaffold; the mass ratio of the polyglycolic acid spinning solution to the polyethylene glycol spinning solution for the inner tube wall of the tubular polymer composite scaffold is 15:1-50:1, and the mass ratio of the polyglycolic acid spinning solution to the polyethylene glycol spinning solution for the outer tube wall of the tubular polymer composite scaffold is 5:1-15:1;
S2: seeding seed cells on the tubular polymer composite scaffold, and culturing for 3 weeks to 3 months in a certain culture condition simulating an arterial pulsatile flow in a body to give an engineering blood vessel composed of the seed cells and an extracellular matrix; and
S3: decellularizing the engineering blood vessel to remove vascular cell components, so as to give the tissue engineering blood vessel composed of the extracellular matrix.

2. The method for preparing the tissue engineering blood vessel for the *in vivo* rapid recellularization according to claim 1, wherein the tubular polymer scaffold has an inner diameter of 1-35 mm, a tube wall thickness of 0.01-3.5 mm, a length of 0.5-120 cm, and a tube wall porosity of 85-99.9%.

3. The method for preparing the tissue engineering blood vessel for the *in vivo* rapid recellularization according to claim 1, wherein the culture condition simulating the arterial pulsatile flow in the body in step S2 is as follows:
firstly, a static *in vitro* culture of 1-10 days; secondly, a culture of 6-8 days in an arterial pulsatile flow at a pressure of 10-90 mmHg; thirdly, a culture in an arterial pulsatile flow at an increased pressure of 90-110 mmHg; and finally, a culture of one week in an arterial pulsatile flow at an increased pressure of 110-160 mmHg.

4. The method for preparing the tissue engineering blood vessel for the *in vivo* rapid recellularization according to claim 1, wherein the decellularization in step S3 comprises the following steps:
treating the engineering blood vessel in a prepared decellularization reagent for 1-3 h at room temperature, and then replacing the decellularization reagent 3-6 times, once every 1-3 h, to give the tissue engineering blood vessel composed of the extracellular matrix;
the decellularization reagent is prepared from 3-[(3-cholamidopropyl)-diethylammonio]-propanesulfonate, disodium edetate (EDTA-2Na), NaCl, NaOH, and sterile deionized water.

5. The method for preparing the tissue engineering blood vessel for the *in vivo* rapid recellularization according to claim 1, wherein the seeding density for the tubular polymer composite scaffold is 0.1-3.0 × 10⁶ cells/centimeter.

6. The method for preparing the tissue engineering blood vessel for the *in vivo* rapid recellularization according to claim 1, wherein the seed cells include, but are not limited to, vascular smooth muscle cells or fibroblasts from an adult human or mammal, vascular smooth muscle cells from an umbilical vein/umbilical artery, and vascular smooth muscle cells or fibroblasts differentiated from a mesenchymal stem cell and an induced pluripotent stem cell.

7. The method for preparing the tissue engineering blood vessel for the *in vivo* rapid recellularization according to claim 1, wherein the seed cells are primary to 10th-passage cells from one or more donors or a cell bank.

## Patentansprüche

1. Verfahren zur Herstellung eines gewebetechnisch hergestellten Blutgefäßes für eine schnelle Rekellularisierung *in vivo,* umfassend:
S1: getrenntes Lösen von Polyglykolsäure und Polyethylenglykol jeweils in einem organischen Lösungsmittel zur Herstellung von Spinnlösungen und Herstellen eines röhrenförmigen Polymerverbundgerüsts durch Elektro pinnen aus den beiden Spinnlösungen in einem bestimmten Verhältnis, wobei das Massenverhältnis der Polyglykolsäure-Spinnlösung zur Polyethylenglykol-Spinnlösung für eine innere Rohrwand des röhrenförmigen Polymerverbundgerüsts höher ist als das Massenverhältnis der Polyglykolsäure-Spinnlösung zur Polyethylenglykol-Spinnlösung für eine äußere Rohrwand des röhrenförmigen Polymerverbundgerüsts; das Massenverhältnis der Polyglykolsäure-Spinnlösung zur Polyethylenglykol-Spinnlösung für die innere Rohrwand des röhrenförmigen Polymerverbundgerüsts beträgt 15:1-50:1, und das Massenverhältnis der Polyglykolsäure-Spinnlösung zur Polyethylenglykol-Spinnlösung für die äußere Rohrwand des röhrenförmigen Polymerverbundgerüsts beträgt 5:1-15:1;
S2: Besäen des röhrenförmigen Polymerverbundgerüsts mit Saatzellen und Kultivieren über 3 Wochen bis 3 Monate unter einer bestimmten Kulturbedingung, die einen arteriellen pulsierenden Fluss im Körper simuliert, um ein gewebetechnisch hergestelltes Blutgefäß zu erhalten, das aus den Saatzellen und einer extrazellulären Matrix besteht; und
S3: Dezellularisieren des gewebetechnisch hergestellten Blutgefäßes zum Entfernen vaskulärer Zellkomponenten, um dadurch das gewebetechnisch hergestellte Blutgefäß zu erhalten, das aus der extrazellulären Matrix besteht.

2. Verfahren zur Herstellung des gewebetechnisch hergestellten Blutgefäßes für die schnelle Rekellularisierung *in vivo* nach Anspruch 1, wobei das röhrenförmige Polymergerüst einen Innendurchmesser von 1-35 mm, eine Rohrwanddicke von 0,01-3,5 mm, eine Länge von 0,5-120 cm und eine Rohrwandporosität von 85-99,9 % aufweist.

3. Verfahren zur Herstellung des gewebetechnisch hergestellten Blutgefäßes für die schnelle Rekellularisierung *in vivo* nach Anspruch 1, wobei die Kulturbedingung, die den arteriellen pulsierenden Fluss im Körper in Schritt S2 simuliert, wie folgt ist:
zunächst eine statische *In-vitro-*Kultur von 1-10 Tagen; anschließend eine Kultur von 6-8 Tagen unter einem arteriellen pulsierenden Fluss bei einem Druck von 10-90 mmHg; danach eine Kultur unter einem arteriellen pulsierenden Fluss bei einem erhöhten Druck von 90-110 mmHg; und schließlich eine einwöchige Kultur unter einem arteriellen pulsierenden Fluss bei einem erhöhten Druck von 110-160 mmHg.

4. Verfahren zur Herstellung des gewebetechnisch hergestellten Blutgefäßes für die schnelle Rekellularisierung *in vivo* nach Anspruch 1, wobei das Dezellularisieren in Schritt S3 folgende Schritte umfasst:
Behandeln des gewebetechnisch hergestellten Blutgefäßes in einem hergestellten Dezellularisationsreagenz für 1-3 h bei Raumtemperatur und anschließendes Ersetzen des Dezellularisationsreagenzes 3-6-mal, jeweils einmal alle 1-3 h, um dadurch das gewebetechnisch hergestellte Blutgefäß zu erhalten, das aus der extrazellulären Matrix besteht;
wobei das Dezellularisationsreagenz aus 3-[(3-Cholamidopropyl)-diethylammonio]-propanesulfonat, Dinatriumedetat (EDTA-2Na), NaCl, NaOH und sterilem deionisiertem Wasser hergestellt ist.

5. Verfahren zur Herstellung des gewebetechnisch hergestellten Blutgefäßes für die schnelle Rekellularisierung *in vivo* nach Anspruch 1, wobei die Besäendichte für das röhrenförmige Polymerverbundgerüst 0,1-3,0 × 10⁶ Zellen/Zentimeter beträgt.

6. Verfahren zur Herstellung des gewebetechnisch hergestellten Blutgefäßes für die schnelle Rekellularisierung *in vivo* nach Anspruch 1, wobei die Saatzellen umfassen, ohne darauf besränkt zu sein, glatte Gefäßmuskelzellen oder Fibroblasten eines erwachsenen Menschen oder Säugetiers, glatte Gefäßmuskelzellen aus einer Nabelvene/Nabelarterie sowie aus mesenchymalen Stammzellen und induzierten pluripotenten Stammzellen differenzierte glatte Gefäßmuskelzellen oder Fibroblasten umfassen.

7. Verfahren zur Herstellung des gewebetechnisch hergestellten Blutgefäßes für die schnelle Rekellularisierung *in vivo* nach Anspruch 1, wobei die Saatzellen Primär- bis Zellen der 10. Passage von einem oder mehreren Spendern oder aus einer Zellbank sind.

## Revendications

1. Procédé de préparation d'un vaisseau sanguin modifié par un tissu à recellularisation rapide *in vivo,* comprenant :
S1: la dissolution de l'acide polyglycolique et du polyéthylène glycol séparément dans un solvant organique pour préparer des solutions de filage, et la préparation d'un échafaudage composite polymère tubulaire par filage électrostatique à partir des deux solutions de filage selon un certain rapport, dans lequel le rapport massique de la solution de filage d'acide polyglycolique à la solution de filage de polyéthylène glycol pour une paroi de tube interne de l'échafaudage composite polymère tubulaire est supérieur au rapport massique de la solution de filage d'acide polyglycolique à la solution de filage de polyéthylène glycol pour une paroi de tube externe de l'échafaudage composite polymère tubulaire ; le rapport massique de la solution de filage d'acide polyglycolique à la solution de filage de polyéthylène glycol pour la paroi de tube interne de l'échafaudage composite polymère tubulaire est de 15:1-50:1, et le rapport massique de la solution de filage d'acide polyglycolique à la solution de filage de polyéthylène glycol pour la paroi de tube externe de l'échafaudage composite polymère tubulaire est de 5:1-15:1 ;
S2: l'ensemencement de cellules germinales sur l'échafaudage composite polymère tubulaire, et la culture pendant 3 semaines à 3 mois dans une certaine condition de culture simulant un flux pulsatile artériel dans un corps pour aboutir à un vaisseau sanguin modifié composé des cellules germinales et d'une matrice extracellulaire ; et
S3: la décellularisation du vaisseau sanguin modifié pour éliminer les composants de cellules vasculaires, de manière à aboutir au vaisseau sanguin modifié tissulaire composé de la matrice extracellulaire.

2. Procédé de préparation du vaisseau sanguin modifié par un tissu à recellularisation rapide *in vivo* selon la revendication 1, dans lequel l'échafaudage polymère tubulaire présente un diamètre interne de 1-35 mm, une épaisseur de paroi de tube de 0,01-3,5 mm, une longueur de 0,5-120 cm, et une porosité de paroi de tube de 85-99,9 %.

3. Procédé de préparation du vaisseau sanguin modifié par un tissu à recellularisation rapide *in vivo* selon la revendication 1, dans lequel la condition de culture simulant l'écoulement pulsatile artériel dans le corps à l'étape S2 est la suivante :
premièrement, une culture *in vitro* statique de 1-10 jours ; deuxièmement, une culture de 6-8 jours dans un flux pulsatile artériel à une pression de 10-90 mmHg ; troisièmement, une culture dans un flux pulsatile artériel à une pression augmentée de 90-110 mmHg ; et enfin, une culture d'une semaine dans un flux pulsatile artériel à une pression augmentée de 110-160 mmHg.

4. Procédé de préparation du vaisseau sanguin modifié par un tissu à recellularisation rapide *in vivo* selon la revendication 1, dans lequel la décellularisation à l'étape S3 comprend les étapes suivantes :
le traitement du vaisseau sanguin modifié dans un réactif de décellularisation préparé pendant 1-3 h à température ambiante, puis le remplacement du réactif de décellularisation 3-6 fois, une fois tous les 1-3 h, pour aboutir au vaisseau sanguin modifié tissulaire composé de la matrice extracellulaire ;
le réactif de décellularisation est préparé à partir de 3-[(3-cholamidopropyl)-diéthylammonio]-propanesulfonate, d'édétate disodique (EDTA-2 Na), de NaCl, de NaOH et d'eau désionisée stérile.

5. Procédé de préparation du vaisseau sanguin modifié par un tissu à recellularisation rapide *in vivo* selon la revendication 1, dans lequel la densité d'ensemencement pour l'échafaudage composite polymère tubulaire est de 0,1-3,0 ×10⁶ cellules/centimètre.

6. Procédé de préparation du vaisseau sanguin modifié par un tissu à recellularisation rapide *in vivo* selon la revendication 1, dans lequel les cellules ensemencées comportent, mais ne sont pas limitées à, des cellules de muscle lisse vasculaire ou des fibroblastes provenant d'un humain ou d'un mammifère adulte, des cellules de muscle lisse vasculaire provenant d'une veine ombilicale/artère ombilicale, et des cellules de muscle lisse vasculaire ou des fibroblastes différenciés provenant d'une cellule souche mésenchymateuse et d'une cellule souche pluripotente induite.

7. Procédé de préparation du vaisseau sanguin modifié par un tissu à recellularisation rapide *in vivo* selon la revendication 1, dans lequel les cellules germes sont des cellules primaires à des cellules de 10ème passage provenant d'un ou de plusieurs donneur(s) ou d'une banque de cellules.
